(19) Europäisches Patentamt
European Patent Office
Office européen des brevets

(11) **EP 4 657 368 A1**

(12) **EUROPEAN PATENT APPLICATION**

(43) Date of publication:
**03.12.2025 Bulletin 2025/49**

(21) Application number: **24179009.6**

(22) Date of filing: **30.05.2024**

(51) International Patent Classification (IPC):
**G06T 7/00** (2017.01)    **G06T 7/11** (2017.01)
**G16H 30/40** (2018.01)

(52) Cooperative Patent Classification (CPC):
**G16H 30/40; G06T 7/11;** G06T 2207/10081;
G06T 2207/20081; G06T 2207/20104;
G06T 2207/30096

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **SCHNELLBÄCHER, Nikolas David**
  **Eindhoven (NL)**
• **NICKISCH, Hannes**
  **Eindhoven (NL)**

(74) Representative: **Philips Intellectual Property &
Standards
High Tech Campus 52
5656 AG Eindhoven (NL)**

(54) **TARGET CONTRAST SETTINGS FOR MEDICAL IMAGES**

(57)    A method and system for preferentially display-ing 2D slices of a 3D medical image according to deter-mined contrast settings. The method comprises gener-ating a file that contains a plurality of target contrast settings and a plurality of position indicators, each associated with a 2D slice of the 3D image. Each target contrast setting enhances the appearance of a target anatomical feature in the associated 2D slice, while each position indicator describes the position of the target anatomical feature in the associated 2D slice. The gen-erated file is then provided to a user interface system configured to display the 2D slices of the 3D image according to the information in the file. Specifically, in the displayed 2D slice, a respective target contrast set-ting is applied to at least a target region in the 2D slice that contains the target anatomical feature, as determined from a respective position indicator.

FIG. 2

**Description**

FIELD OF THE INVENTION

The present invention relates to the field of image processing, and in particular, to methods of determining contrast settings for 2D slices of 3D images.

BACKGROUND OF THE INVENTION

In diagnostic radiology, 3D images are acquired to obtain representative views of a patient's internal anatomy. Notable examples of radiological techniques include CT, MRI and ultrasound imaging. The obtained 3D images are subsequently analyzed by a medical professional (e.g., a radiographer) in order to diagnose a medical condition of the patient. Such analysis often involves scrolling through different 2D slices of the 3D image to view different parts of the patient's anatomy, some of which may be indicative of the patient's condition.

Controlling the contrast between different parts/objects in the 2D slices is important for identifying relevant features. Dependent on the imaging technique used, and the exact anatomy being probed, predefined contrast settings may be provided for initially displaying the 2D slices. However, these contrast settings may often need to be adjusted to better suit the exact situation, requiring manual adjustment from the medical professional. This slows the rate at which the medical professional can analyze the acquired image, resulting in a delayed diagnosis for the patient, and additionally increased fatigue for the medical professional.

There is thus a desire for an automated method for (pre-)determining contrast settings for 3D medical images that enhances the appearance of desired anatomical features in the 3D images, and/or a system for displaying 2D slices of the 3D images according to the determined contrast settings.

SUMMARY OF THE INVENTION

The invention is defined by the claims.

According to examples in accordance with an aspect of the invention, there is provided a computer-implemented method for processing 2D slices of a 3D image comprising medical imaging data of a subject. The computer-implemented method comprises generating a file containing a plurality of target contrast settings and a plurality of position indicators. The file is generated by, for each 2D slice of the 3D image: identifying, in the 2D slice, a target region containing a representation of a target anatomical feature in the 3D image, wherein the target region is smaller than the 2D slice; determining a target contrast setting for the target region responsive to the target region and/or the target anatomical feature; and storing the target contrast setting and a position indicator, indicative of a position of the target region in the 2D slice, in the file.

The present invention provides a method for processing 2D slices of a 3D image for the purposes of displaying the 3D image on a user interface system. Specifically, the invention relates to 3D images comprising 3D medical imaging data (e.g., CT, MR, ultrasound, PET, SPECT or OCT imaging data) of a subject (e.g., a patient). In other words, the 3D image is a volume comprising (a portion of) the subject's body that at least contains a target anatomical feature/object that a user (e.g., a medical professional) wishes to observe/study, e.g., for the purposes of diagnosing a medical condition, planning a medical treatment or performing a medical intervention.

The 3D image is displayed as 2D slices/images (i.e., 2D cuts through the 3D volume of the 3D image) on the user interface system. More specifically, the 2D slices of the 3D image are displayed one at a time, and a user may switch/scroll between different 2D slices to observe different cuts through the subject's body, and thus different cuts/views of the target feature. Through processing of the 3D image prior to displaying on the user interface system, the invention enables each 2D slice to be displayed using a target or preferred contrast setting, applied at least to a target region, that enhances the visibility of the target feature.

In particular, the present invention overcomes the problem of a user having to manually change contrast settings as they scroll/switch between different 2D slices. More specifically, each 2D slice may require different contrast settings to advantageously view/identify the target feature in the 2D slice, which (in normal circumstances) the user must change manually. The proposed invention instead provides a process that allows for automated adjustment of the contrast settings applied to a 2D slice, such that the target feature is always displayed using a target or preferred contrast setting. This may save the user time by not requiring them to repeatedly adjust contrast settings as they scroll through different 2D slices, and may be particularly useful for inexperienced users who have not developed an instinctual ability for adjusting contrast settings to advantageously view a target feature. There is therefore a reduced risk of a potentially dangerous pathology being overlooked or missed by a clinician.

Specifically, the proposed method comprises generating a file that contains contrast information that a user interface system may utilize to display 2D slices of the 3D image. More precisely, a file is generated that contains a plurality of target contrast settings that describe preferential contrast settings for the different 2D slices. Additionally, the file contains a plurality of position indicators that describe (i.e., indicate) where in each 2D slice the respective target contrast setting should (at least) be applied to. In other words, each position indicator is indicative of a minimum area in the corresponding 2D slice to apply the target contrast setting to. Accordingly, the target contrast setting may, in certain examples, be applied to the entirety of the 2D slice, or a part (i.e., not all) of the 2D slice that encompasses the area described by the position indicator. In some examples, the target contrast setting may be applied to only the area of the 2D slice described by the position indicator, or a predefined scalar multiple of said area.

In order to generate the file, a target region (i.e., an area in a 2D slice) is identified for each 2D slice that contains at least a representation (e.g., a cross-section) of a target anatomical feature comprised within the 3D image. A target contrast setting is then determined that displays said representation of the target anatomical feature in the target region.

The target anatomical feature may comprise an organ (of the subject) or a particular portion of an organ, or a particular portion of the subject's body. Alternatively (or additionally) the target anatomical feature may comprise a particular feature formed on an organ (or more generally formed on/in the subject's body) such as a lesion and/or an anomaly.

Finally, for each 2D slice, the determined target contrast setting, along with the position indicator that indicates a position of the target region in the 2D slice, is stored in the file. The file may then be provided to a user interface system for displaying 2D slices of the 3D image.

In some examples, for each 2D slice of the 3D image, the target region may be identified using a classification algorithm or a segmentation algorithm. Such algorithms may comprise machine-learning algorithms that have been trained to identify particular anatomical features/objects in medical images.

In some examples, for each 2D slice of the 3D image, the target contrast setting may be determined responsive to historical data associated with the target anatomical feature. Such historical data may comprise past 3D images and/or medical imaging data (from the same subject and/or from different subjects) containing the target anatomical feature, for which all or some of the 2D slices are associated with corresponding contrast settings for ideally viewing the target anatomical feature, as determined by a medical professional.

The historical data may be accessed from a database (e.g., a PACS database) and used to generate a lookup table. Specifically, the lookup table may describe preferential contrast settings for different 2D slices through a 3D volume of a subject's body. Target contrast settings for each of the 2D slices of the present 3D image may thus be determined with reference to the lookup table.

Furthermore, for each 2D slice of the 3D image, the target contrast setting may be determined from a machine-learning model configured to process the target region and/or the target anatomical feature, wherein the machine-learning model is trained using the historical data associated with the target anatomical feature.

In some examples, one or more target contrast settings of the plurality of target contrast settings may comprise a non-linear monotonic function.

Additionally, or alternatively, the computer-implemented method may further comprise applying a smoothing process to the plurality of target contrast settings, wherein the smoothing process comprises adjusting one or more target contrast settings such that, when a portion of the 3D image is displayed, discontinuities between the contrast of the target regions of adjacent 2D slices in the 3D image are attenuated.

In other words, following the smoothing process, the target contrast settings vary smoothly across the plurality of target contrast settings. This may help prevent "flickering" effects when scrolling through the different 2D slices that may be caused by erratic or noisy variations between target contrast settings.

Also provided is a user interface system for displaying 2D slices of a 3D image comprising medical imaging data of a subject. The user interface system is configured to receive a file containing contrast information associated with a target anatomical feature in the 3D image and display a 2D slice of the 3D image, wherein, in response to the contrast information comprising a plurality of target contrast settings and a plurality of positions indicators, and dependent on the displayed 2D slice, a respective target contrast setting is applied at least to a corresponding target region, determined from a respective position indicator, in the displayed 2D slice.

In some examples, the respective target contrast setting may be applied to a region-of-interest in the displayed 2D slice, wherein the region-of-interest contains the corresponding target region. For example, the region-of-interest may comprise a circular or a polygonal shape, such as a box, that is centered on the target region.

Furthermore, the user interface system may be further configured to, in response to the contrast information comprising a plurality of target contrast settings and a plurality of positions indicators, and dependent on the displayed 2D slice, apply a weighted contrast setting, being a weighted average between the respective target contrast setting and a further contrast setting applied to a remainder of the displayed 2D slice, to a border region surrounding the region-of-interest, wherein said weighting changes dependent on a perpendicular distance away from an edge of the region-of-interest.

In particular, the weighting may be configured such that, across the border region, the weighted contrast setting varies from being predominantly the same as the target contrast setting at the edge of the region-of-interest, to being predominantly the same as the further contrast setting at an edge of the border region furthest from the region-of-interest. Such an approach avoids the case of a sharp change in contrast at the edge of the region-of-interest.

In some examples, the target anatomical feature may comprise a lesion and/or an anomaly. In such cases, the user interface system may be further configured to provide a user-perceptible priority indicator that indicates one or more priority 2D slices of the 3D image, being 2D slices that display the lesion and/or the anomaly, wherein the one or more priority 2D slices are determined responsive to the corresponding target region in each 2D slice.

In other words, the priority indicator, as indicated on the user interface system, communicates to a user the preferable 2D slices for viewing the lesion and/or the anomaly. Such a feature may save the user time, e.g., as the user is no longer required to search through different 2D slices to find an ideal view of the lesion and/or the anomaly.

Additionally, or alternatively, in response to the contrast information not comprising the plurality of target contrast settings and the plurality of position indicators, a default contrast setting, associated with the target anatomical feature, may be applied to the displayed 2D slice. In this way, the user interface system may be "backwards compatible" with old/historic files, i.e., files that have not been generated using the proposed method, and thus do not contain the plurality of target contrast settings and position indicators.

In most cases, the old files will instead contain the default contrast setting. However, in cases where the file does not contain the default contrast setting (e.g., the file does not contain any contrast settings) the user interface system may instead retrieve the default contrast setting from a memory or a database/server or from default settings stored by the user interface system.

In some examples, the file may further contain the 3D image. In this way, the user interface system may only receive a single file that contains all the data required for displaying 2D slices of the 3D image with target/preferential contrast settings, i.e., the 3D image, the plurality of target contrast settings and the plurality of position indicators.

Also provided is a computer program product comprising computer program code means which, when executed on a computing device having a processing system, cause the processing system to perform all of the steps of the method according to the above disclosure.

Further provided is a processing system for processing 2D slices of a 3D image comprising medical imaging data of a subject. The processing system is configured to generate a file containing a plurality of target contrast settings and a plurality of position indicators. The file is generated by, for each 2D slice of the 3D image: identifying, in the 2D slice, a target region containing a representation of a target anatomical feature in the 3D image, wherein the target region is smaller than the 2D slice; determining a target contrast setting for the target region responsive to the target region and/or the target anatomical feature; and storing the target contrast setting and a position indicator, indicative of a position of the target region in the 2D slice, in the file.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a medical image analysis system;
Fig. 2 shows an example 2D slice from a 3D image; and
Fig. 3 shows a flowchart illustrating a proposed method.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for processing a 3D image. Specifically, the method comprises processing 2D slices of the 3D image to determine a preferred (i.e., target) contrast setting for each 2D slice of the 3D image. Accordingly, when a particular 2D sliced is displayed (e.g., on a screen) a corresponding preferred contrast setting is applied to the displayed 2D slice that results in the appearance of a target feature in the 2D slice being enhanced, i.e., being more easily perceived by the human eye.

In particular, the 3D image (preferably) comprises medical imaging data of a subject (e.g., a patient) and the target feature is (preferably) a target anatomical feature within the subject, such as an organ or a feature comprised by/on an organ. The method also (more precisely) comprises generating a file that contains the target contrast settings (i.e., a plurality of target contrast settings) and further information related to the position of the target feature in each 2D slice.

Consequently, the generated file may be provided to a user interface system capable of displaying the 2D slices of the 3D image. In particular, on receiving the file, the user interface is configured to display a 2D slice of the 3D image according to the target contrast settings in the file, thereby enhancing the appearance of a target anatomical feature in the displayed 2D slice. By enhancing the appearance of the target anatomical feature in the displayed 2D slices, a user (e.g., a medical/healthcare professional) can more easily identify and study the target anatomical feature, which may speed up diagnosis of a medical condition of the subject.

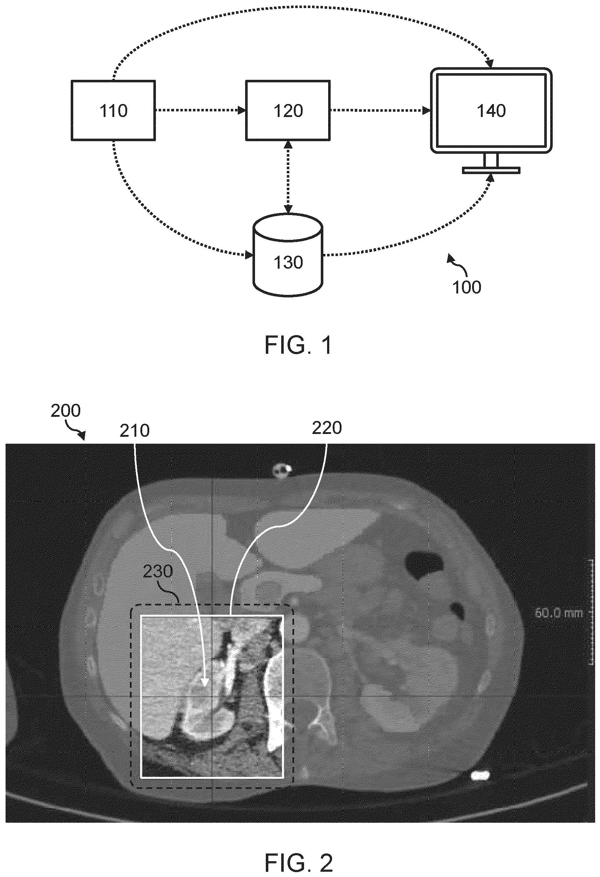

Fig. 1 schematically illustrates a medical image analysis system 100 in which the present invention can be employed. The medical image analysis system 100 comprises a medical imaging system 110, a processing system 120, a database 130, and a user interface system 140.

The function of the medical image analysis system 100 is to obtain, process, and display medical images of a subject for the purposes of diagnosing or assessing a medical condition of the subject (e.g., a disease, an illness, an injury, etc.) and/or for guiding a medical intervention. The medical images provided by the medical image analysis system 100 can be analyzed by a user to diagnose or assess the medical condition. Specifically, the user may analyze the medical images by interacting with (i.e., operating) the user interface system 140 which is configured to display the medical images.

The same user may also operate other elements of the medical image analysis system 100, or the other elements may be operated by a different user (e.g., dependent on an availability and/or a specialization of the users). Furthermore, it will be recognized that the different elements of the medical image analysis system 100 may not be located substantially close to each other (e.g., not in the same room, not in the same building). Accordingly, the different elements of the medical image analysis system 100 may be configured to communicate (e.g., send instructs, transfer information, etc.) using one or more wireless communication protocols and/or via one or more servers.

The medical imaging system 110 is configured to obtain, collect, or otherwise acquire, medical imaging data of the subject. Preferably, said medical imaging data is 3D medical imaging data that forms, or may otherwise be reconstructed into, (e.g., by processing the 3D medical imaging data using the processing system 120 or the medical imaging system 110 itself) a 3D medical image that represents a 3D volume comprising a portion of the subject's body. Accordingly, the medical imaging system 110 is preferably a medical device capable of acquiring 3D medical imaging data. Suitable examples of such medical devices include CT, MRI, ultrasound, PET, SPECT and OCT scanners/machines.

To be more specific, the 3D image obtained from the medical imaging data is a 3D dataset representing a 3D volume. The 3D dataset comprises a discrete number of regularly arranged (e.g., in rows, columns and layers) data points, each data point being a data value (i.e., a numerical value) that is associated with a position (e.g., an (x, y, z) coordinate) in the 3D volume. In most cases, the data values are signal intensity values associated with the medical imaging technique used to acquire the medical imaging data, and therefore dependent on the properties/characteristics of the materials/-substances comprised within the 3D volume (e.g., air, bone, soft tissue, etc.). For example, in the case of CT imaging, the data values may be CT-numbers representing the attenuation properties of different materials/objects in the 3D volume.

The 3D image is hence a visual representation of the 3D dataset, where each data point of the 3D dataset becomes a pixel/voxel of the 3D image. In particular, the data value of each data point can be mapped to a corresponding intensity/brightness/color for the pixels in the 3D image, according to appropriate color scales and/or contrast settings.

Owing to the discrete nature of the 3D dataset, the 3D image can be segmented into a series of 2D slices/images that correspond to 2D layers of the 3D dataset, i.e., discrete 2D datasets "stacked" along a particular axis of the 3D dataset. Each 2D slice therefore represents a slice/cut through the 3D volume represented by the 3D image, e.g., a slice through the subject. The 2D slices can hence provide an internal view of the subject's body, an example of which can be seen in Fig. 2.

Following acquisition (and potential processing) of the medical imaging data, the medical imaging system 110 is configured to provide the medical imaging data and/or the 3D image to one or more other elements of the medical image analysis system 100. For example, the medical imaging system 110 may provide the medical imaging data to the processing system 120 for the purposes of obtained the 3D image (e.g., by reconstruction). Alternatively, the medical imaging system 110 may provide the 3D image to the processing system 120 in cases when the medical imaging system 110 is configured to obtain the 3D image from the medical imaging data. As will be later disclosed, following receiving or obtaining the 3D image, the processing system 120 is configured to process the 3D image to determine preferred/target contrast settings for the 2D slices of the 3D image.

It will be understood that providing the medical imaging data and/or the 3D image to an element of the medical image analysis system 100 comprises providing, to said element, a file containing the medical imaging data and/or the 3D image. As will be later disclosed, said file(s) may contain only the medical imaging data or the 3D image, or may comprise further information/data.

The medical imaging system 110 may also provide the medical imaging data and/or the 3D image to the database 130. More specifically, the medical imaging system 110 may store the medical imaging data and/or the 3D image in the database 130. Once stored in the database 130, the medical imaging data and/or the 3D image may be retrieved (e.g., at a later time) by the processing system 120 and/or the user interface system 140.

In particular, the database 130 may be a PACS database that allows for the confidential storage and retrieval of medical images and imaging data between different medical devices and different medical institutions.

The medical imaging system 110 may also provide the 3D image to the user interface system 140. Particularly, the user interface system 140 is configured to display (e.g., on a screen of the user interface system 140) 2D slices of the received 3D image. More precisely, the user interface system 140 displays the 2D slices one at a time (i.e., only one 2D slice is displayed by the user interface system 140 at a particular point in time). By operating the user interface system 140, a user may switch/change, or otherwise control, which 2D slice of the 3D image is displayed by the user interface system 140.

For completeness, the user interface system 140 may also receive/retrieve the 3D image from the processing system 120 or the database 130.

The volume represented by the 3D image will preferably comprise/contain a target anatomical feature (e.g., an organ of the subject) that a user wishes to observe and/or analyze. More precisely, the medical imaging system 110 will preferably be configured to acquire medical imaging data from a portion of the subject's body that comprises the target anatomical feature. In particular, it will be understood that the target anatomical feature may be associated with a medical condition of the subject that the user wishes to diagnose or assess through observation and/or analysis of the target anatomical feature.

More generally, the target anatomical feature is any internal anatomical feature and/or object of the subject which may be relevant for diagnosing or assessing a medical condition of the subject.

The user may therefore view the target anatomical feature (or more precisely a representation of the target anatomical feature) using the user interface system 140. Specifically, the user may view cuts/slices through the target anatomical feature from the 2D slices displayed by the user interface system 140. By operating the user interface system 140, the user may switch/scroll between different 2D slices, thus observing different cuts through the target anatomical feature. Accordingly, the user may diagnose or assess (or at least rule out potential options for) the medical condition of the subject from viewing different cuts through the target anatomical feature.

For clarity, where a subject's anatomy (such as a target anatomical feature) is said to be in, observable, or otherwise comprised by, a (displayed) 2D slice, it will be understood to mean the 2D slice comprises a representation of the subject's anatomy, i.e., the 2D slice does not comprise the actual/physical anatomy but is merely a graphical representation of the anatomy. In other words, when a user views the 2D slice (which comprises, or is otherwise derived, from medical imaging data from the subject) the user is viewing a representation of the anatomy of the subject and not the physical anatomy itself.

Fig. 2 shows an example 2D slice 200 of a 3D image acquired using CT imaging. Specifically, the 2D slice 200 shows (a representation of) a (perpendicular) cut through the torso of a subject/patient, which reveals the internal anatomy of the subject/patient. A target anatomical feature 210, here corresponding to a kidney of the subject/patient, is visible in the 2D slice (or more precisely a cross-section of the target anatomical feature 210 can be seen). From the observed cut/cross-section through the target anatomical feature 210, a user may be able to diagnose or assess a medical condition of the subject/patient. Alternatively, the user may require a different view of the target anatomical feature 210 (and therefore a different 2D slice of the 3D image) to observe a particular detail on or of the target anatomical feature 210 that will enable them to diagnose or assess the (condition of the) subject/patient.

The intensity/brightness/color of each pixel in the 2D slice 200 is dependent on the data value (in this case the CT-number) associated with each pixel/data point and a corresponding color scale and contrast setting for the 2D slice 200 that maps each data value (and hence pixel) to a corresponding color and/or brightness.

In the present context, the color scale defines a range, sequence and/or gradient of colors that the pixels are mapped to. In the example of Fig. 2, the color scale is a "grayscale" that defines a color gradient from black at one extreme to white at the other, with corresponding shades of gray in-between. Specifically, pixels with low data values, i.e., CT-numbers, (corresponding to low amounts of attenuation) are assigned darker (i.e., blacker) colors, while pixels with high data values (corresponding to high amounts of attenuation) are assigned brighter (i.e., whiter) colors. Other possible color scales will be known to those skilled in the art.

On top of this, the contrast setting provides further specifics to the mapping between the data value of each pixel and colors of the color scale, i.e., to which color each data value corresponds. At a minimum, the contrast setting defines a contrast window, being a data value range described by a minimum value $V_{min}$, and a maximum value $V_{max}$, over which to apply the color scale. Specifically, following with the current example, pixels with a data value less than or equal to $V_{min}$ are assigned the color black, while pixels with a data value greater than or equal to $V_{max}$ are assigned the color white. Pixels with a data value in between $V_{min}$ and $V_{max}$ (i.e., intermediate values) are hence assigned a gradient color of the color scale (i.e., different shades of gray).

In the simplest case, intermediate values may be assigned/mapped to colors of the color scale using a linear relationship. In some cases, however, this mapping may use a non-linear monotonic relationship, such as an exponential, logarithmic, hyperbolic, or parabolic relationship. This effectively modulates the contrast sensitivity of different parts of the contrast window according to the relationship used. For example, a small variation between data values within one portion of the contrast window may result in a large change in visible contrast/color, whereas a small variation between data values within another portion of the contrast window may result in a negligible change in visible contrast/color. This may be desirable if a user wishes to enhance the level of detail within a region of the 2D slice that comprises only a small variation in data values, but still wants to maintain noticeable contrast between objects that comprise a large variation in data values.

The contrast setting thus defines a data value range over which the color/brightness of corresponding pixels vary. Accordingly, the contrast setting affects what features/objects in the 2D slice are visible (and additionally a corresponding level of detail that can be observed within those features). For example, in the 2D slice 200, the contrast setting used/applied results in internal organs of the subject (including the target anatomical feature 210) becoming visible to the human eye.

Note, however, for areas of the 2D slice 200 outside of the region 220 (which will be discussed later) many of the organs appear homogeneous (i.e., are a single color and/or lack detail within their cross-section) and/or are faint compared to the surrounding tissue. Adjustment of the contrast setting applied to the 2D slice 200 may result in the appearance of those organs becoming enhanced, i.e., easier to see with the human eye and comprising a greater amount of detail.

Accordingly, for a particular 2D slice, there may exist a contrast setting (or a range of contrast settings) for which a target anatomical feature is advantageously displayed in the 2D slice, i.e., it is easier to observe (and therefore easier to analyze) compared to using other contrast settings. In other words, the 2D slice may have a preferred/target contrast setting for observing the target anatomical feature.

Returning to Fig. 1, in the current state of the art, the 3D image provided to the user interface system 140 may not be provided with contrast settings (in which case the user interface system 140 may display the 2D slices of the 3D image using preconfigured contrast settings of the user interface system 140) or may be provided with one or more predefined contrast settings, associated with the target anatomical feature in the 3D slice and/or with the imaging technique used (e.g., by the medical imaging system 110) to acquire the 3D image. In both cases, neither contrast settings may correspond to preferential/target contrast settings.

As mentioned previously, a user analyzing a 3D image of a subject may need to scroll between different 2D slices in order to accurately diagnose or assess a medical condition of the subject. Additionally, different 2D slices may be associated with different contrast settings. The user may therefore be required to manually change/adjust the contrast settings applied to a displayed 2D slice in order to enhance the appearance of a target anatomical feature. This may result in the user spending an increased amount of time analyzing the 3D image due to needing to repeatedly adjust the contrast settings applied to different displayed 2D slices.

It would therefore be desirable for the 3D image to be provided to the user interface system 140 with target contrast settings for each 2D slice of the 3D image, such that each 2D slice displayed by the user interface system 140 is automatically displayed according to the target contrast settings.

In accordance with the present invention, the processing system 120 is configured to determine target contrast settings for each 2D slice of the received/obtained 3D image. In particular, the target contrast settings enhance the appearance of (at least) a target anatomical feature in each 2D slice when displayed according to the target contrast settings. Accordingly, the processing system 120 may provide the determined target contrast settings (among other relevant information) to the user interface system 140 in order for the 2D slices to be displayed by the user interface system 140 using the target contrast settings.

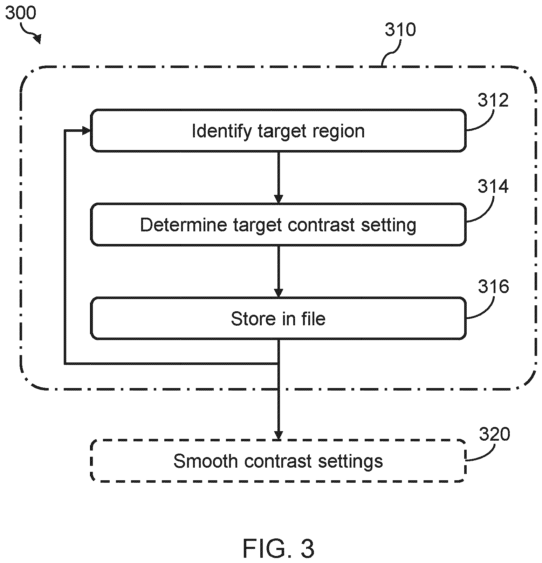

Fig. 3 shows a flowchart outlining a method 300 to be performed by the processing system 120. In particular, the method 300 relates to the processing of 2D slices of the 3D image to determine a target contrast setting for each of the 2D slices that enhances the appearance of a target anatomical feature in the 2D slices.

More specifically, the method 300 comprises generating 310 a file that contains a plurality of target contrast settings, where each target contrast setting of the plurality of target contrast settings is associated with a respective 2D slice of the 3D image. The generated file also contains a plurality of position indicators associated with a position of the target anatomical feature in each of the 2D slices.

In particular, step 310 may first comprise generating an empty file for which the plurality of target contrast settings (and plurality of position indicators) determined by the method 300 can be stored in. Alternatively, step 310 may comprise receiving a file (either empty or containing further information) to store the plurality of target contrast settings in, as generated prior to the method 300.

The process 310 of generating the file comprises performing a plurality of steps 312, 314 and 316 for each 2D slice of the 3D image. In some cases, generating 310 the file may comprise performing the plurality of steps 312, 314 and 316 iteratively, i.e., performing all of steps 312, 314 and 316 for a single 2D slice before performing steps 312, 314 and 316 for another (i.e., subsequent) 2D slice. In other cases, generating 310 the file may comprise performing multiple instances of the plurality of steps 312, 314 and 316 for all of the 2D slices in parallel before performing the next step. In yet other cases, generating 310 the file may comprise performing a predefined number of instances of the plurality of steps 312, 314, 316 for a subset of the 2D slices, comprising only some of the 2D slices, and iteratively repeating this process for different and distinct subsets of the 2D slices until all 2D slices have been processed.

Step 312 comprises identifying, in a 2D slice of the 3D image, a target region that contains (at least) the target anatomical feature. More precisely, the target region is an area (in the 2D slice) that contains/covers the cross-section of the target anatomical feature present in the 2D slice. Additionally, it will be understood that the target region is smaller than the 2D slice, e.g., no greater than 50% of the size of the 2D slice or no greater than 25% of the size of the 2D slice. In other words, identification of the target region comprises identifying a part of the 2D slice.

In particular, the target region will preferably contain the entirety of the representation of the target anatomical feature in the 2D slice. The target region therefore indicates the part of the 2D slice that is most relevant to the target anatomical feature.

In some examples, the target region may be identified using a classification (i.e., object detection) algorithm or a segmentation algorithm. Preferably, such algorithms may make use of machine-learning models that have been trained to identify particular anatomical features in medical images. Accordingly, the classification and segmentation algorithms may be responsive to the target anatomical feature, i.e., the specific anatomical feature that is to be located/identified in the 2D slice.

The above algorithms may function (e.g., in the case of classification algorithms) by identifying shapes in the 2D slice that resemble expected shapes for the cross-section of the target anatomical feature, and/or (e.g., in the case of segmentation algorithms) by analyzing the data values of different pixels in the 2D slice. Accordingly, the algorithms may process the data values of the 2D slice in order to identify the target region, e.g., by identifying the boundaries of different objects/features in the 2D slice, and/or by identifying pixels/data values associated with the target anatomical feature.

In the case of classification algorithms, the resulting target region may comprise a polygonal shape, such as a rectangle (i.e., a bounding box), that fully contains the target anatomical feature (as well as some adjacent areas of the 2D slice). Alternatively, in the case of segmentation algorithms, the resulting target region may comprise a complex shape that matches the shape of the target anatomical feature, i.e., the target region only contains pixels of the 2D slice that have been identified to belong to the target anatomical feature.

One suitable example of an appropriate algorithm is the "You-only-look-once" algorithm, e.g., described by Redmon, Joseph, et al. "You only look once: Unified, real-time object detection." Proceedings of the IEEE conference on computer vision and pattern recognition 2016.

The target region is therefore defined by a shape/area that fully contains/encloses the target anatomical feature in the 2D slice. Similarly, the target region is defined by a position in the 2D slice, i.e., where the shape described by the target region must be positioned in the 2D slice in order to contain the target anatomical feature. Such a position may be described, or otherwise indicated, by a position indicator. In some cases, the position indicator may also describe/indicate the shape/area of the target region, such that the target region for the 2D slice may be fully inferred from the position indicator.

For example, when the target region comprises a regular shape, the position indicator may comprise a set of coordinates that describe a position of the center of the shape in the 2D slice. Additionally, the position indicator may comprise one or more characteristic lengths that describe a size of the shape (e.g., side lengths). As an alternative, the size and shape of the target region may be predefined, such that the position indicator need not identify the size/shape of the target region.

Alternatively, when the target region comprises a complex shape, the position indicator may comprise a "mask", being a (2D) dataset comprising a plurality of data points equal in number to, and associated with, the pixels in the 2D slice. Each data point may then be assigned a number that indicates whether the associated pixel in the 2D slice forms part of the target region. For example, a data point with a value of "0" may indicate that the associated pixel is not part of the target region, whereas a data point with a value of "1" may indicate that the associated pixel is part of the target region.

Step 314 comprises determining a target contrast setting for a 2D slice. In particular, step 314 comprises determining the target contrast setting for at least a target region in the 2D slice as identified in step 312. The target contrast setting is hence determined for (and is therefore responsive to) a part of the 2D slice rather than the entirety of the 2D slice. More specifically, the target contrast setting is determined responsive to the target region (i.e., the data values of the pixels contained within the target region) and/or the target anatomical feature (i.e., the specific anatomy contained within the target region).

In some examples, determining the target contrast setting may comprise processing the target region. In particular, the target contrast setting may be determined by processing/analyzing the data values associated with the target region to determine a data value range, i.e., a minimum and a maximum data value that the data values of the target region are between. Accordingly, the target contrast setting may be determined responsive to the data value range. It has been previously explained how a contrast window may be defined using a minimum value and a maximum value. The determined minimum data value and the determined maximum data value may define the data range value of this contrast window.

Alternatively (or additionally) the target contrast setting may be determined responsive to historical data associated with the target anatomical feature. In the present context, historical data corresponds to 3D medical images acquired from previous/other subjects, or acquired from the same subject at a previous/past time. Accordingly, the historical data may be associated with the target anatomical feature if it contains the same or a similar anatomical feature as the target anatomical feature, e.g., the historical data contains a particular organ as given by the target anatomical feature, or a particular detail on an organ.

Furthermore, the historical data will preferably be associated with preferential/preset contrast settings for viewing the target anatomical feature in the historical data. More precisely, for some or all of the 2D slices of the historical data that contain a representation of the target anatomical feature, there may preferably be corresponding contrast settings for ideally observing the target anatomical feature in the 2D slices when displayed, e.g., by a user interface system. Such preset contrast settings may have been (manually) determined by a medical professional, i.e., the preset contrast settings may correspond to contrast settings that the medical professional deemed appropriate for viewing the target anatomical feature.

The historical data may thus provide a reference against which to determine target contrast settings for the present 2D slices. For example, the historical data and associated preset contrast settings may be used to generate a lookup table. The lookup table may describe/indicate target/preferential contrast settings for viewing the target anatomical feature (and potentially other target anatomical features) in different 2D slices. Specifically, the lookup table may map a particular 2D slice to a corresponding contrast setting. Such mapping may take into account a position of the 2D slice in the 3D image (i.e., the specifics (e.g., position, direction) of the cut through the subject's body represented by the 2D slice) and therefore the expected representation of the target anatomical feature in the 2D slice. Accordingly, the lookup table may be used to determine target contrast settings for the 2D slices of the 3D image.

The lookup table may be generated by a processing system (e.g., the processing system 120) responsive to historical data retrieved from a database (e.g., the database 130). Alternatively, the lookup table may be pre-generated and retrieved by the processing system from the database when determining the target contrast settings.

Alternatively, the historical data may be used to train a machine-learning model that is capable of providing the target contrast settings. Specifically, the machine-learning model may be trained using a supervised-learning approach comprising providing the historical data as inputs to the machine-learning model, with the associated preset contrast settings being an expected output to compare against the actual outputs of the machine-learning model.

More precisely, the machine-learning model may process the 2D slices of the historical data (or parts of the 2D slices associated with the target anatomical feature) to determine (i.e., output) estimated target contrast settings. The machine-learning model may then be iteratively adjusted, dependent on the difference between the estimated target contrast settings and the preset contrast settings, until the outputs of the machine-learning model become substantially similar to the preset contrast settings.

Accordingly, the machine learning model becomes configured to process new 2D slices (e.g., the 2D slices of the 3D image), and preferably the target region of the 2D slice, to determine corresponding target contrast settings, e.g., by processing target regions in the 2D slices and/or the target anatomical feature of the 2D slices.

In some cases, the target contrast setting determined in step 314 may comprise a non-linear monotonic function.

Step 316 comprises storing the target contrast setting determined in step 314 in the (generated) file. Additionally, the position indicator associated with the 2D slice (for which the target contrast setting was determined for) is also stored in the file. In particular, the target contrast setting and the position indicator are stored in such a way that they are known (or can be determined) to be associated with each other and with the 2D slice. In other words, the 2D slice, the target contrast setting, and the position indicator, form a set of "linked" information relevant to displaying the 2D slice to enhance the appearance of the target anatomical feature.

Accordingly, following performing steps 312, 314 and 316 for all 2D slices of the 2D image, a file is generated that contains a plurality of target contrast settings and a plurality of position indicators. In some cases, the file may only contain the plurality of target contrast settings and the plurality of position indicators, such as when the file was first generated as an empty file at the start of the method 300.

The file may contain information/data other than the plurality of target contrast settings and the plurality of position indicators. For example, the file may also contain the 3D image. In such examples, the 3D image may have been copied or transferred from its original file to the newly generated file containing the plurality of target contrast settings and the plurality of position indicators.

The act of generating the file in step 310 may comprise storing the plurality of target contrast settings and the plurality of position indicators in the file containing the 3D image. In other words, step 316 may comprise storing the target contrast setting and the position indicator in the file containing the 3D image. In particular, the plurality of target contrast settings and the plurality of position indicators may be stored as metadata in the file containing the 3D image. In this way, each target contrast setting and position indicator pair may be more easily linked/associated with the corresponding 2D slice in the 3D image.

In accordance with the above examples, the file(s) containing the plurality of target contrast settings and the plurality of position indicators and/or the 3D image may be DICOM file(s). For example, the plurality of target contrast settings and the plurality of position indicators may be stored in (and/or associated with) one or more tags of the DICOM file(s). In some examples, the tags may comprise one or more private (i.e., custom) tags defined for the present disclosure. Said file(s) may therefore conform to an industry/medical standard so that it/they is/are compatible with existing frameworks, data processing and/or transferring systems, and devices.

Following generating the file according to step 310, the method 300 may comprise applying a smoothing process 320 to the plurality of target contrast settings in the file. In the present context, the smoothing process refers to adjustment/modification of one or more target contrast settings of the plurality of target contrast settings such that large differences/changes between adjacent contrast settings (referring to contrast settings associated with adjacent 2D slices) are reduced or removed. In other words, discontinuities between adjacent contrast settings are attenuated. In particular, the "smoothing" of the smoothing process does not refer to smoothing within a particular target contrast setting, but smoothing across/between different target contrast settings.

To provide further context, it will be recognized that, in step 314, the target contrast setting is determined solely from the present associated 2D slice, and is therefore independent of other 2D slices in the 3D image. Accordingly, a scenario can be envisioned in which adjacent target contrast settings in the plurality of target contrast settings vary rapidly and/or erratically. Such variation may be in the values of $V_{min}$ and $V_{max}$, and/or in the intermediate (value) mapping functions, between adjacent target contrast settings. Accordingly, when scrolling/switching between 2D slices displayed on a user interface system, the appearance of the 2D slices (as set by the target contrast settings) may change rapidly, causing a flickering effect.

The smoothing process of step 320 thus helps to prevent or reduce flickering effects by increasing the (perceived) smoothness between target contrast settings of the plurality of target contrast settings. Put another way, adjacent target contrast settings are made more similar., i.e., differences in $V_{min}$, $V_{max}$, and intermediate mapping functions between adjacent target contrast settings are reduced. Accordingly, target contrast settings may continue to be modified using the smoothing process until a measure of difference between adjacent target contrast settings falls below a predefined threshold.

Corresponding adjustment(s) made to a particular target contrast setting may hence be responsive to one or more differences and/or one or more rates of change between the particular target contrast setting and one or more adjacent and/or nearby (e.g., next-adjacent) target contrast settings. Such differences and/or rates of change may be determined by processing one or more of $V_{min}$, $V_{max}$, and intermediate mapping functions for the relevant target contrast settings.

In accordance with the above disclosure, the method 300 (when performed by the processing system 120 of Fig. 1) thus outputs a file that contains a plurality of target contrast settings and a plurality of position indicators, each associated with a 2D slice of the 3D image. The file may then be provided to the user interface system 140 for the purposes of displaying 2D slices of the 3D image. Specifically, the file may be provided to the user interface system 140 by the processing system 120 directly, or via the database 130, i.e., the user interface system 140 may retrieve the file from the database 130.

More generally, the user interface system 140 is configured to receive or retrieve a file containing (at least) contrast information (e.g., contrast settings) associated with a target anatomical feature in a 3D image. Accordingly, it will also be understood that the user interface system 140 is also configured to access the contrast information in the file.

In some cases, the file may contain both the contrast information and the corresponding 3D image. Put another way, the user interface system 140 may receive or retrieve (e.g., from the processing system 120 or the database 130) a single file that contains all the relevant information required to display 2D slices of the 3D image.

Alternatively, the user interface system 140 may receive the 3D image and the contrast information as separate files. In such cases, the user interface system 140 may receive each file from the same element of the medical image analysis system 100 (e.g., both from the processing system 120 or both from the database 130). Instead, the user interface system 140 may receive each file from different elements of the medical image analysis system 100 (e.g., the 3D image from the medical imaging system 110 and the contrast information from the processing system 120).

Following with the above examples, the user interface system 140 may be configured to only display 2D slices of a 3D image after receiving or retrieving a file containing relevant contrast information. Furthermore, in response to receiving the 3D image but no contrast information, the user interface system 140 may be configured to retrieve (e.g., from the database 130) a file containing contrast information relevant to the 3D image. For example, the file containing the 3D image may also contain an indicator that directs the user interface system 140 to a particular file in the database 130. Alternatively, the user interface system 140 may retrieve a "default" file from the database 130 containing default contrast information (e.g., default contrast setting(s)) associated with the target anatomical feature in the 3D image. In the present context, default contrast information/setting(s) may refer to an industry/medical standard as known in the art, i.e., well-known contrast setting(s) associated with a target anatomical feature. The user interface system 140 may hence be preconfigured to know, or know where to retrieve, the default contrast information.

Accordingly, upon receiving/retrieving the file containing the contrast information, the user interface system 140 is configured to display a 2D slice of the 3D image according to the contrast information in the file. Particularly, in response to the contrast information comprising a plurality of target contrast settings and a plurality of position indicators (i.e., the file containing the contrast information was generated by the processing system 120 according to the method 300 of Fig. 3) the user interface system 140 is configured to display the 2D slice with a respective target contrast setting applied to at least a corresponding target region in the displayed 2D slice. More specifically dependent on the displayed 2D slice, a respective target contrast setting associated with the displayed 2D slice is applied to at least the corresponding target region, determined from a respective position indicator associated with the displayed 2D slice.

Furthermore, it will be understood that, should a user operating the user interface system 140 change the 2D slice displayed by the user interface system 140 from an initial 2D slice to a further (i.e., different) 2D slice, the further 2D slice will similarly be displayed according to a target contrast setting associated with the further 2D slice, applied at least to a further target region determined from a position indicator associated with the further 2D slice.

Of course, it will be appreciated that not all 2D slices may be associated with target contrast settings. Such 2D slices may be controlled to have default contrast settings.

In some cases, however, the contrast information may not comprise a plurality of target contrast settings and a plurality of position indicators. In other words, the file received/retrieved by the user interface system 140 may not have been generated by the processing system 120 according to the method 300 of Fig. 3. In such cases, it is expected that the contrast information will most likely comprise default contrast setting(s) associated with the target anatomical feature and/or default settings of the user interface system 140. Accordingly, the 2D slice (displayed by the user interface system 140) may be displayed according to the default contrast setting(s), e.g., a single default contrast setting may be applied to all/any 2D slice(s) displayed by the user interface system 140.

For completeness, it is also recognized that the contrast information may comprise neither target contrast settings or default contrast settings, and may instead comprise some other form of contrast information. The user interface system 140 should hence not be considered to be limited to only displaying 2D slices according to target and default contrast settings, but may also be configured to display 2D slices using contrast information/settings determined using a process/paradigm different to that of the present disclosure.

It will also be understood that a user operating the user interface system 140 may also be able to manually change (e.g., override) the contrast settings applied to the displayed 2D slice using one or more controls of the user interface system 140. Furthermore, when the received contrast information comprises a plurality of target contrast settings and a plurality of position indicators, the user may also be permitted to switch from using the target contrast settings to either of default contrast settings or manual contrast settings, and vice versa.

As mentioned previously, when displaying 2D slices according to the target contrast settings, the user interface system 140 is configured to apply a respective target contrast setting to at least a corresponding target region in the 2D slice. In particular, the target region, as identified in step 312 of the method 300 of Fig. 3, contains the entirety of the target anatomical feature present in the displayed 2D slice, the appearance of which becomes enhanced by the use of the respective target contrast setting.

In some examples, the user interface system 140 may be configured to also apply the respective target contrast setting to a region-of-interest in the displayed 2D slice, being a portion/area of the displayed 2D slice that contains the target region. In other words, the user interface system 140 may be configured to apply the respective target contrast setting to the target region and to a portion of the 2D slice surrounding the target region, as defined by the region-of-interest.

Fig. 2 shows an example of the above disclosure. Specifically, a target contrast setting is applied to a region-of-interest 220 in the 2D slice 200. The appearance/contrast of objects/features within the region-of-interest 220, including the target anatomical feature 210, are thus enhanced compared to those outside of the region-of-interest 220.

In the present example, the region-of-interest 220 has a rectangular shape, however, it will be appreciated that the region-of-interest may take the form of any circular, polygonal, or irregular shape. The region-of-interest may also be user defined, e.g., set and/or drawn by a user operating the user interface system 140. Alternatively, the region-of-interest may be predefined, i.e., defined/determined prior to the user interface system 140 displaying the 2D slice. For example, the region-of-interest may be determined at the same time as the target region. In other words, step 312 of the method 300 of Fig. 3 may comprise identifying a target region and a region-of-interest responsive to the target region.

As seen in Fig. 2, applying the target contrast setting to only the region-of-interest 220 results in a sharp change in the appearance/contrast of the 2D slice 200 at the edge of the region-of-interest 220. To avoid or reduce this effect, the user interface system 140 may be configured to apply a dynamic/variable contrast setting to a border region 230 surrounding the region-of-interest 220 that "blends" the appearance of the region-of-interest 220 into the remainder of the 2D slice 200. More precisely, said blending results in a smooth transition between the target contrast setting of the region-of-interest 220 and the contrast setting of the remainder of the 2D slice 200 (i.e., a further contrast setting).

The border region 230 represents a loop of fixed width/thickness that bounds (i.e., surrounds) the region-of-interest 220. The dynamic contrast setting applied to the border region 230 thus acts as a buffer between the region-of-interest 220 and the remainder of the 2D slice 200 that reduces harsh/sudden changes in perceived contrast.

An example dynamic contrast setting that may achieve this desired effect is a weighted contrast setting. Specifically, the weighted contrast setting is a weighted average between the target contrast setting applied to the region-of-interest and the further contrast setting applied to the remainder of the 2D slice 200, where said weighting is also dependent on a perpendicular distance away from an edge of the region-of-interest 220. An equation describing such a weighted contrast setting is provided below:

$$C_w = \left(\frac{W - D}{W}\right)C_t + \left(\frac{D}{W}\right)C_i, \tag{1}$$

where $C_w$, $C_t$ and $C_i$ are the weighted contrast setting, target contrast setting, and further contrast setting, respectively, W is the width of the border region 230, and D is the perpendicular distance from an edge of the region-of-interest 220. From the provided equation, it can be seen how the weighted contrast setting changes from being predominantly equal to the target contrast setting near the edge of the region-of-interest 220, to being predominantly equal to the further contrast setting near the edge of the border region 230. The contrast setting applied to the border region 230 thus changes gradually from the target contrast setting to the further contrast setting, thereby providing the desired smoothing effect.

It has been previously disclosed that the target anatomical feature may comprise an organ and/or a particular feature/detail comprised by or on an organ (or simply a portion of the subject's anatomy). In more specific examples, the target anatomical feature may (also) comprise a lesion (e.g., a cut, an injury) and/or an anomaly (e.g., an abscess, a tumor). In such examples, the lesion and/or the anomaly may be formed on, or comprised by, an organ of the subject (or more generally a portion of the subject). Furthermore, the lesion and/or the anomaly may only form part of the target anatomical feature, e.g., the target anatomical feature may comprise the entire organ that comprises the lesion and/or the anomaly.

In most cases, however, the lesion and/or the anomaly may represent a key feature of the target anatomical feature, i.e., the part of the target anatomical feature most important for diagnosing a condition of the subject. 2D slices that contain/display the lesion and/or the anomaly may thus be of high priority to a user of the user interface system 140.

Accordingly, the user interface system 140 may be configured to provide a user-perceptible priority indicator that indicates (to the user of the user interface system 140) one or more priority 2D slices of the 3D image. In particular, the one or more priority 2D slices are 2D slices of the 3D image that either contain or display a representation of the lesion and/or the anomaly. Consequently, the user may purposefully change the 2D slice displayed by the user interface system 140 to one of the one or more priority 2D slices in order to the view the lesion and/or the anomaly.

The one or more priority 2D slices may be determined responsive to the target region in each 2D slice of the 3D image. More specifically, a 2D slice of the 3D image may be determined to be a priority 2D slice by processing the target region in the 2D slice to determine whether it contains and/or preferentially shows the lesion and/or the anomaly. With reference to Fig. 3, this may be performed as part of step 312 of the method 300, i.e., after identifying the target region. In other words, the one or more priority 2D slices may be determined by the processing system 120. In such examples, one or more indicators/markers may be provided in the file that communicate to the user interface system 140 which of the 2D slices of the 3D image are priority 2D slices. Alternatively, the one or more priority 2D slices may be determined by the user interface system 140 itself.

In some examples, the user interface system 140 may be configured to automatically display one of the one or more priority 2D slices, e.g., as the initial 2D slice of the 3D image displayed by the user interface system 140. The priority 2D slice automatically displayed by the user interface system 140 may be the priority 2D slice considered to be of highest priority, e.g., the priority 2D slice that shows the lesion and/or the anomaly the most clearly out of all the one or more priority 2D slices. Such a measure of priority may be determined by processing the target region in each 2D slice.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

Note, though the herein provided examples relate primarily to the use of 3D images/datasets, those skilled in the art will recognize that the present disclosure may also apply to 4D datasets, e.g., comprising a time dimension. For example, the 4D dataset may comprise a series of 3D images (representing a portion of the subject's body) measured at different times. Accordingly, target contrast settings may be determined for each 2D slice of each 3D image of the 4D dataset. Furthermore, when viewing 2D slices from a 4D dataset on a user interface system, a user may scroll between 2D slices within a single 3D image, but also between 2D slices between 3D images (i.e., scrolling along a spatial dimension and a time dimension). The user may thus view changes over time within different 2D slices.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method (300) for processing 2D slices (200) of a 3D image comprising medical imaging data of a subject, the computer-implemented method comprising:

   generating a file (310) containing a plurality of target contrast settings and a plurality of position indicators by, for each 2D slice of the 3D image;
   identifying (312), in the 2D slice, a target region containing a representation of a target anatomical feature (210) in the 3D image, wherein the target region is smaller than the 2D slice;
   determining (314) a target contrast setting for the target region responsive to the target region and/or the target anatomical feature; and
   storing (316) the target contrast setting and a position indicator, indicative of a position of the target region in the 2D slice, in the file.

2. The computer-implemented method of claim 1, wherein, for each 2D slice of the 3D image, the target region is identified using a classification algorithm or a segmentation algorithm.

3. The computer-implemented method of any one of claims 1 or 2, wherein, for each 2D slice of the 3D image, the target contrast setting is determined responsive to historical data associated with the target anatomical feature.

4. The computer-implemented method of claim 3, wherein, for each 2D slice of the 3D image, the target contrast setting is determined from a machine-learning model configured to process the target region and/or the target anatomical feature, wherein the machine-learning model is trained using the historical data associated with the target anatomical feature.

5. The computer-implemented method of any one of claims 1 to 4, wherein, one or more target contrast settings of the plurality of target contrast settings comprise a non-linear monotonic function.

6. The computer-implemented method of any one of claims 1 to 5, further comprising applying a smoothing process (320) to the plurality of target contrast settings, wherein the smoothing process comprises adjusting one or more target contrast settings such that a measure of difference between target contrast settings associated with adjacent 2D slices in the 3D image is reduced.

7. A user interface system (140) for displaying 2D slices (200) of a 3D image comprising medical imaging data of a subject, the user interface system being configured to:

   receive a file containing contrast information associated with a target anatomical feature (210) in the 3D image; and
   display a 2D slice of the 3D image, wherein, in response to the contrast information comprising a plurality of target contrast settings and a plurality of position indicators, and dependent on the displayed 2D slice, a respective target contrast setting is applied at least to a corresponding target region, determined from a respective position indicator, in the displayed 2D slice.

EP 4 657 368 A1

8. The user interface system of claim 7, wherein the respective target contrast setting is applied to a region-of-interest (220) in the displayed 2D slice, wherein the region-of-interest contains the corresponding target region.

9. The user interface system of claim 8, further configured to, in response to the contrast information comprising a plurality of target contrast settings and a plurality of positions indicators, and dependent on the displayed 2D slice: apply a weighted contrast setting, being a weighted average between the respective target contrast setting and a further contrast setting applied to a remainder of the displayed 2D slice, to a border region (230) surrounding the region-of-interest, wherein said weighting changes dependent on a perpendicular distance away from an edge of the region-of-interest.

10. The user interface system of any one of claims 7 to 9, wherein the target anatomical feature comprises a lesion and/or an anomaly.

11. The user interface system of claim 10, further configured to provide a user-perceptible priority indicator that indicates one or more priority 2D slices of the 3D image, being 2D slices that display a representation of the lesion and/or the anomaly, wherein the one or more priority 2D slices are determined responsive to the corresponding target region in each 2D slice.

12. The user interface system of any one of claims 7 to 11, wherein, in response to the contrast information not comprising the plurality of target contrast settings and the plurality of position indicators, a default contrast setting, associated with the target anatomical feature, is applied to the displayed 2D slice.

13. The user interface system of any one of claims 7 to 12, wherein the file further contains the 3D image.

14. A computer program product comprising computer program code means which, when executed on a computing device having a processing system, cause the processing system to perform all of the steps of the method according to any of claims 1 to 6.

15. A processing system (120) for processing 2D slices (200) of a 3D image comprising medical imaging data of a subject, the processing system being configured to:

generate a file (310) containing a plurality of target contrast settings and a plurality of position indicators by, for each 2D slice of the 3D image;
identifying (312), in the 2D slice, a target region containing a representation of a target anatomical feature (210) in the 3D image, wherein the target region is smaller than the 2D slice;
determining (314) a target contrast setting for the target region responsive to the target region and/or the target anatomical feature; and
storing (316) the target contrast setting and a position indicator, indicative of a position of the target region in the 2D slice, in the file.

14

FIG. 1

FIG. 2

FIG. 3

Europäisches
Patentamt

European
Patent Office

Office européen
des brevets

# EUROPEAN SEARCH REPORT

**Application Number**

EP 24 17 9009

## DOCUMENTS CONSIDERED TO BE RELEVANT

| Category | Citation of document with indication, where appropriate, of relevant passages | Relevant to claim |
|---|---|---|
| X | US 2020/311912 A1 (KNOPLIOCH JEROME [FR] ET AL) 1 October 2020 (2020-10-01) | 1-5,7-15 |
| Y | * paragraph [0021] - paragraph [0057]; figures 1-5 * | 6 |
| | ----- | |
| Y | US 2021/090257 A1 (BHATIA PARMEET SINGH [US] ET AL) 25 March 2021 (2021-03-25) | 6 |
| A | * paragraph [0100] - paragraph [0165] * | 1-5,7-15 |
| | ----- | |

**CLASSIFICATION OF THE APPLICATION (IPC)**

INV.
G06T7/00
G06T7/11
G16H30/40

**TECHNICAL FIELDS SEARCHED (IPC)**

G06T
G16H

The present search report has been drawn up for all claims

| Place of search | Date of completion of the search | Examiner |
|---|---|---|
| The Hague | 14 November 2024 | Lauritzen, Jan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 17 9009

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2024

| Patent document cited in search report | | | Publication date | Patent family member(s) | | | Publication date |
|---|---|---|---|---|---|---|---|
| US 2020311912 | A1 | | 01-10-2020 | CN | 111768343 | A | 13-10-2020 |
| | | | | EP | 3716202 | A1 | 30-09-2020 |
| | | | | JP | 7194143 | B2 | 21-12-2022 |
| | | | | JP | 2020179159 | A | 05-11-2020 |
| | | | | US | 2020311912 | A1 | 01-10-2020 |
| | | | | US | 2021295512 | A1 | 23-09-2021 |
| US 2021090257 | A1 | | 25-03-2021 | CN | 112529834 | A | 19-03-2021 |
| | | | | EP | 3796210 | A1 | 24-03-2021 |
| | | | | US | 2021090257 | A1 | 25-03-2021 |

EPO FORM P0459

For more details about this annex : see Official Journal of the European Patent Office, No. 12/82